# EUROPÄISCHE PATENTANMELDUNG

(11) **EP 3 736 594 A1**
(43) Veröffentlichungstag der Anmeldung: **11.11.2020**
(21) Anmeldenummer: 19173755.0
(22) Anmeldetag: 10.05.2019
(51) Int. Cl.: G01R 33/563, G01R 33/36, A61B 5/00

(54) **MAGNETRESONANZ-ELASTOGRAPHIE-VORRICHTUNG MIT EINER MAGNETRESONANZ-EINHEIT UND EINER ELASTOGRAPHIE-EINHEIT**

(71) Anmelder: Siemens Healthcare GmbH, 91052 Erlangen (DE)
(72) Erfinder: Kreher, Sabrina, 91094 Langensendelbach (DE); Petsch, Markus, 91056 Erlangen (DE)

(57) **Zusammenfassung**

Die Erfindung geht aus von einer Magnetresonanz-Elastographie-Vorrichtung (10) umfassend:
- eine Magnetresonanz-Einheit (11), die einen Grundmagneten (13), eine Gradientenspuleneinheit (15) und eine Hochfrequenzspuleneinheit (17) aufweist, und
- eine Elastographie-Einheit (50) mit einem Vibrationsapplikator (51), der eine Vibrationserzeugereinheit (52) aufweist, und einer Kopplungseinheit (53) zum Koppeln der Elastographie-Einheit (50) mit der Magnetresonanz-Einheit (11), wobei die Elastographie-Einheit (50) eine Antriebseinheit (55) zum Erzeugen eines Antriebmoments für die Vibrationserzeugereinheit (52) aufweist, wobei die Antriebseinheit (55) magnetresonanzkompatibel ausgebildet ist.

## Beschreibung

Die vorliegende Erfindung betrifft eine Magnetresonanz-Elastographie-Vorrichtung mit einer Magnetresonanz-Einheit und einer Elastographie-Einheit. Die Magnetresonanz-Einheit weist einen Grundmagneten, der zur Erzeugung eines Magnetfelds vorgesehen ist, eine Gradientenspuleneinheit, die zur Erzeugung von Magnetfeldgradienten vorgesehen ist, und eine Hochfrequenzspuleneinheit, die zu einer Einstrahlung von Magnetresonanzsequenzen ausgelegt ist, auf. Die Elastographie-Einheit weist einen Vibrationsapplikator, der eine Vibrationserzeugereinheit aufweist, und eine Kopplungseinheit zum Koppeln der Elastographie-Einheit mit der Magnetresonanz-Einheit, auf.

Tumorgewebe und gesundes Gewebe, insbesondere Gewebe, das tumorfrei ist, weisen unterschiedliche Vibrationseigenschaften und/oder ein unterschiedliches Anregungsverhalten bei einer Vibrationsanregung auf. Die diagnostische Bildgebung nutzt bei der Elastographie die unterschiedlichen Vibrationseigenschaften und/oder das unterschiedliche Anregungsverhalten zwischen den unterschiedlichen Gewebearten, insbesondere einem gesunden Gewebe und einem Tumorgewebe. Dieses unterschiedliche Verhalten kann mittels der Magnetresonanzbildgebung in einem Magnetresonanz-Elastogramm dargestellt werden.

Der vorliegenden Erfindung liegt insbesondere die Aufgabe zugrunde, eine einfache Anbindung einer Elastographie-Einheit an eine Magnetresonanz-Einheit bereitzustellen. Die Aufgabe wird durch die Merkmale des unabhängigen Anspruchs gelöst. Vorteilhafte Ausgestaltungen sind in den Unteransprüchen beschrieben.

Die Erfindung geht aus von einer Magnetresonanz-Elastographie-Vorrichtung, die eine Magnetresonanz-Einheit und eine Elastographie-Einheit umfasst. Die Magnetresonanz-Einheit weist einen Grundmagneten, eine Gradientenspuleneinheit und eine Hochfrequenzspuleneinheit auf. Die Elastographie-Einheit weist einen Vibrationsapplikator, der eine Vibrationseinheit aufweist, und eine Kopplungseinheit zum Koppeln der Elastographie-Einheit mit der Magnetresonanz-Einheit. Erfindungsgemäß weist die Elastographie-Einheit eine Antriebseinheit zum Erzeugen eines Antriebmoments für die Vibrationserzeugereinheit auf, wobei die Antriebseinheit magnetresonanzkompatibel ausgebildet ist.

Die Magnetresonanz-Elastographie-Vorrichtung ist dazu ausgelegt, unterschiedliche Vibrationseigenschaften und/oder ein unterschiedliches Anregungsverhalten von unterschiedlichen Gewebearten, wie beispielsweise von gesundem, tumorfreien Gewebe und Tumorgewebe in einem Magnetresonanz-Elastogramm darzustellen. Hierzu wird mittels der Elastographie-Einheit ein zu untersuchender Bereich des Patienten mittels Vibrationen angeregt. Das unterschiedliche Verhalten wird mittels der Magnetresonanzbildgebung in einem Magnetresonanz-Elastogramm dargestellt.

Die Magnetresonanz-Elastographie-Vorrichtung weist hierzu die Magnetresonanz-Einheit auf. Die Magnetresonanz-Einheit weist einen Scannereinheit und einen von der Scannereinheit bevorzugt zylinderförmig umgebenen Patientenaufnahmebereich auf. Innerhalb des Patientenaufnahmebereichs befindet sich eine Patient oder ein zu untersuchender Bereich des Patienten für eine Magnetresonanz-Elastographie-Untersuchung. Die Scannereinheit weist den Grundmagneten, die Gradientenspuleneinheit und die Hochfrequenzspuleneinheit auf. Ein von dem Grundmagneten erzeugtes Magnetfeld ist insbesondere innerhalb des Patientenaufnahmebereichs, bevorzugt innerhalb eines Field of Views (FOVs) des Patientenaufnahmebereichs, möglichst homogen und konstant ausgebildet. Die Gradientenspuleneinheit ist zu einer Erzeugung von Magnetfeldgradienten während einer Magnetresonanzbildgebung ausgebildet, wobei die Magnetfeldgradienten für eine Ortskodierung während der Magnetresonanzbildgebung verwendet werden. Die Hochfrequenzspuleneinheit ist während einer Magnetresonanzmessung zu einem Einstrahlen von hochfrequenten Magnetresonanzsequenzen in den Patientenaufnahmebereich, insbesondere in das FOV, der Magnetresonanz-Einheit vorgesehen.

Der Vibrationsapplikator der Elastographie-Einheit ist insbesondere zu einem Generieren von Vibrationen und/oder Druckwellen und zu einem Übertragen der generierten Vibrationen und/oder Druckwellen an den Patienten und/oder auf den Patienten ausgelegt. Bevorzugt wird hierbei der Vibrationsapplikator in einer Nähe zu dem Patienten, insbesondere in einer Nähe des zu untersuchenden Bereichs des Patienten, positioniert. Beispielsweise wird hierbei der Vibrationsapplikator an den zu untersuchenden Bereich des Patienten angelegt. Während einer Magnetresonanz-Elastographie-Untersuchung befindet sich somit der Vibrationsapplikator der Elastographie-Einheit innerhalb des Patientenaufnahmebereichs, insbesondere innerhalb des FOVs, und damit innerhalb des Magnetfelds der Magnetresonanz-Einheit.

Des Weiteren weist die Elastographie-Einheit eine Kopplungseinheit zu einem Koppeln der Elastographie-Einheit mit der Magnetresonanz-Einheit. Die Kopplungseinheit ist bevorzugt zu Verbindung der Elastographie-Einheit mit der Magnetresonanz-Einheit vorgesehen. Insbesondere ist die Kopplungseinheit zu einem Austausch von Steuerungssignalen zwischen der Elastographie-Einheit und der Magnetresonanz-Einheit ausgelegt. Des Weiteren kann die Kopplungseinheit bevorzugt auch zu einer elektrischen Anbindung der Elastographie-Einheit mit der Magnetresonanz-Einheit ausgelegt sein, so dass die Elastographie-Einheit über die Kopplungseinheit mit elektrischer Energie versorgt werden kann. Mittels der Kopplungseinheit kann vorteilhaft eine Bilddatenerfassung der Magnetresonanz-Einheit mit der Elastographie-Einheit während einer Magnetresonanz-Elastographie-Untersuchung abgestimmt werden. Zudem kann somit auch die Elastographie-Einheit von einer Steuerungseinheit der Magnetresonanz-Einheit angesteuert werden und somit die Magnetresonanz-Elastographie-Untersuchung mittels einer einzigen Steuerungseinheit gesteuert werden. Die Kopplungseinheit kann dabei eine kabelgebundene Kopplungseinheit umfassen. Zudem ist auch eine kabellose und/oder eine drahtlose Kopplungseinheit jederzeit möglich.

Unter einer magnetresonanzkompatibel ausgebildeten Antriebseinheit soll dabei insbesondere verstanden werden, dass die Antriebseinheit weder eine Magnetresonanzmessung beeinflusst noch mit dem Magnetfeld und/oder einem Gradientenfeld und/oder einem Hochfrequenzfeld der Magnetresonanz-Einheit interagiert. Diese Ausgestaltung der Erfindung ermöglicht es, dass die Antriebseinheit besonders nahe an der Vibrationserzeugereinheit angeordnet werden kann. Damit kann auch ein kurzer Übertragungsweg zu einem Übertragen eines von der Antriebseinheit generierten Antriebsmoments auf die Vibrationserzeugereinheit bereitgestellt werden und auf lange und störanfällige Übertragungswege zum Übertragen des Antriebsmoments von der Antriebseinheit auf die Vibrationserzeugereinheit vorteilhaft verzichtet werden. Zudem kann derart auch eine Antriebseinheit mit einer geringen Leistung zur Verfügung gestellt werden. Dies ermöglicht auch eine besonders kostengünstige Herstellung und/oder Bauweise der Elastographie-Einheit.

Ein weiterer Vorteil ist, dass die Elastographie-Einheit hierdurch besonders kompakt und bauraumsparend gebaut werden kann. Dies ermöglicht auch eine einfache Bedienung und/oder Handhabung der Elastographie-Einheit durch ein medizinisches Bedienpersonal während einer Magnetresonanz-Elastographie-Untersuchung. Zudem kann auch aufgrund der kompakten Bauweise ein hoher Patientenkomfort während einer Magnetresonanz-Elastographie-Messung bereitgestellt werden.

In einer vorteilhaften Weiterbildung der Erfindung kann es vorgesehen sein, dass die Antriebseinheit eine Piezo-Antriebseinheit umfasst. Unter einer Piezo-Antriebseinheit soll hierbei insbesondere eine Antriebseinheit verstanden werden, die den piezoelektrischen Effekt zur Erzeugung und/oder Generierung eines Antriebsmoments nutzt. Dabei wird bevorzugt eine Bewegung und/oder ein Antriebsmoment aufgrund einer Gleit- oder Haftreibung zwischen einem feststehenden Teil (Stator) und einem bewegten Teil (Rotor) erzeugt und/oder generiert. Vorteilhafterweise können dabei Piezo-Antriebseinheiten auch eine durch piezoelektrische Festkörperaktoren erzeugte, insbesondere resonante, Schwingung des Stators zur Erzeugung der Bewegung und/oder des Antriebsmoments nutzen. Besonders vorteilhaft ist hierbei, dass Piezo-Antriebseinheiten kein Magnetfeld zur Generierung und/oder Erzeugung einer Bewegung und/oder eines Antriebsmoments benötigen und somit auch keine Interaktion und/oder Beeinflussung mit dem Magnetfeld der Magnetresonanz-Einheit erfolgen kann.

In einer vorteilhaften Weiterbildung der Erfindung kann es vorgesehen sein, dass die Antriebseinheit innerhalb des Vibrationsapplikators angeordnet ist. Es kann hierdurch eine direkte Übertragung des Antriebsmoments von der Antriebseinheit auf die Vibrationserzeugereinheit erfolgen. Zudem kann aufgrund dieser Ausbildung vorteilhaft auf eine lange Übertragungseinheit verzichtet werden, wie beispielsweise eine Übertragungswelle zum Übertragen des Antriebsmoments von außerhalb des Patientenaufnahmebereichs zur der Vibrationserzeugereinheit. Insbesondere kann derart auf eine starre Übertragungseinheit zwischen der Antriebseinheit und dem Vibrationsapplikator vorteilhaft verzichtet werden. Dies ermöglicht zudem eine besonders kompakte Bauweise der Elastographie-Einheit. Zudem kann hierdurch auch ein besonders flexibler Einsatz und/oder eine besonders flexible Verwendung des Vibrationsapplikators für eine Magnetresonanz-Elastographie-Untersuchung bereitgestellt werden.

In einer vorteilhaften Weiterbildung der Erfindung kann es vorgesehen sein, dass die Antriebseinheit innerhalb der Kopplungseinheit angeordnet ist. Hierdurch kann vorteilhaft der Vibrationsapplikator der Elastographie-Einheit besonders kompakt und besonders leicht gebaut werden. Damit kann insbesondere ein hoher Patientenkomfort bereitgestellt werden und vorteilhaft ein Druck auf den Patienten während einer Magnetresonanz-Elastographie-Untersuchung auf aufgrund eines Anlegens des Vibrationsapplikators an dem Patienten reduziert werden.

In einer vorteilhaften Weiterbildung der Erfindung kann es vorgesehen sein, dass die Elastographie-Einheit ein Kraftübertragungselement aufweist, das ein von der Antriebseinheit erzeugtes Antriebsmoment auf die Vibrationserzeugereinheit überträgt. Hierdurch kann vorteilhaft eine direkte Kraftübertragung und/oder eine direkte Übertragung des Antriebsmoments von der Antriebseinheit auf die Vibrationserzeugereinheit erfolgen. Ist die Antriebseinheit innerhalb des Vibrationsapplikators angeordnet, so ist bevorzugt auch das Kraftübertragungselement innerhalb des Vibrationsapplikators angeordnet, so dass das Kraftübertragungselement besonders kompakt und leicht gebaut werden kann. Ist dagegen die Antriebseinheit außerhalb des Vibrationsapplikators angeordnet, wie beispielsweise innerhalb der Kopplungseinheit, so ist bevorzugt das Kraftübertragungselement zwischen der Antriebseinheit und des Vibrationsapplikator angeordnet. Besonders vorteilhaft umfasst das Kraftübertragungselement eine Übertragungswelle. Zudem kann das Kraftübertragungselement in einer alternativen Ausgestaltung der Erfindung auch weitere, dem Fachmann als sinnvoll erscheinende Elemente aufweisen, wie beispielsweise Getriebeelemente und/oder Zahnräder usw.

In einer vorteilhaften Weiterbildung der Erfindung kann es vorgesehen sein, dass das Kraftübertragungselement eine flexible Übertragungswelle aufweist. In diesem Zusammenhang soll unter einer flexiblen Übertragungswelle insbesondere eine biegsame und/oder biegbare Übertragungswelle, insbesondere eine Biegewelle, verstanden werden. Eine derartige Übertragungswelle kann vorteilhaft eingesetzt werden, wenn ein Übertragungsweg zwischen zwei Punkten, wie insbesondere zwischen der Antriebseinheit und der Vibrationserzeugereinheit, einen gekrümmten Weg und/oder Abbiegungen aufweist und keinen geraden Weg aufweist. Insbesondere kann derart eine besonders kompakte und bauteilesparende Elastographie-Einheit bereitgestellt werden, da auf zusätzliche Bauteile, wie beispielsweise auf zusätzliche Kraftübertragungselemente und/oder Getriebeelemente, verzichtet werden kann. Zudem kann derart auch ein hoher Patientenkomfort während einer Magnetresonanz-Elastographie-Untersuchung vorteilhaft erreicht werden, da sowohl ein kompakter und gewichtsparender Vibrationsapplikator als auch eine kompakte und bauteilesparende Übertragungswelle bereitgestellt werden kann. Zudem kann die Elastographie-Einheit besonders kostengünstig hergestellt werden.

In einer vorteilhaften Weiterbildung der Erfindung kann es vorgesehen sein, dass die Kopplungseinheit ein Steckerelement aufweist, das mit einem Spulensteckerelement der Magnetresonanz-Einheit zur Aufnahme eines Spulensteckers einer lokalen Hochfrequenzspule kompatibel ausgebildet ist. Bevorzugt ist hierbei das Steckerelement der Kopplungseinheit entsprechend und/oder gleichartig zu einem Steckerelement einer lokalen Hochfrequenzspule ausgebildet. Derart kann eine besonders einfache Kopplung und/oder Anbindung der Elastographie-Einheit an die Magnetresonanz-Einheit erfolgen. Insbesondere kann derart auch die Elastographie-Einheit direkt mittels einer Steuerungseinheit der Magnetresonanz-Einheit angesteuert werden und somit durch die Steuerungseinheit der Magnetresonanz-Einheit auch eine Elastographie-Anwendung mittels der Elastographie-Einheit mit einer Magnetresonanzbildgebung mittels der Magnetresonanz-Einheit vorteilhaft abgestimmt werden. Mittels der Kopplungseinheit, insbesondere des Steckerelements der Kopplungseinheit, der Elastographie-Einheit kann zudem vorteilhaft eine Energieversorgung der Elastographie-Einheit während einer Magnetresonanz-Elastographie-Untersuchung erfolgen. Das Spulensteckerelement der Magnetresonanz-Einheit ist bevorzugt an einer Patientenlagerungsvorrichtung, auf der der Patient für eine MagnetresonanzBildgebung positioniert ist, angeordnet. Vorzugsweise umfasst die Patientenlagerungsvorrichtung zwei oder mehr Spulensteckerelemente, so dass gleichzeitig eine lokale Hochfrequenzspule und die Elastographie-Einheit während einer Magnetresonanz-Elastographie-Untersuchung verwendet werden können. Zudem kann derart auch eine besonders sichere und platzsparende Kopplung und/oder Anbindung der Elastographie-Einheit an die Magnetresonanz-Einheit bereitgestellt werden, da die Kopplung direkt an der Patientenlagerungsvorrichtung erfolgen kann.

In einer alternativen Ausbildung der Erfindung kann die Kopplungseinheit auch ein separat zu einem Spulenstecker ausgebildetes Steckerelement aufweisen. Vorzugsweise weist hierzu die Magnetresonanz-Einheit und/oder die Patientenlagerungsvorrichtung ebenfalls ein weiteres Steckerelement auf, das komplementär zu dem Steckerelement der Kopplungseinheit der Elastographie-Einheit ausgebildet ist.

In einer vorteilhaften Weiterbildung der Erfindung kann es vorgesehen sein, dass die Elastographie-Einheit eine elektrische Verbindung zwischen der Kopplungseinheit und dem Vibrationsapplikator aufweist. Die elektrische Verbindung kann eine drahtlose und/oder eine kabellose Verbindung umfassen. Besonders vorteilhaft jedoch umfasst die elektrische Verbindung zwischen der Kopplungseinheit und dem Vibrationsapplikator eine kabelgebundene elektrische Verbindung.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanz-Elastographie-Vorrichtung mit einer Magnetresonanz-Einheit und einer Elastographie-Einheit in einer schematischen Darstellung,
- Fig. 2: ein erstes Ausführungsbeispiel der Elastographie-Einheit in einer schematischen Darstellung und
- Fig. 3: ein zweites Ausführungsbeispiel der Elastographie-Einheit in einer schematischen Darstellung.

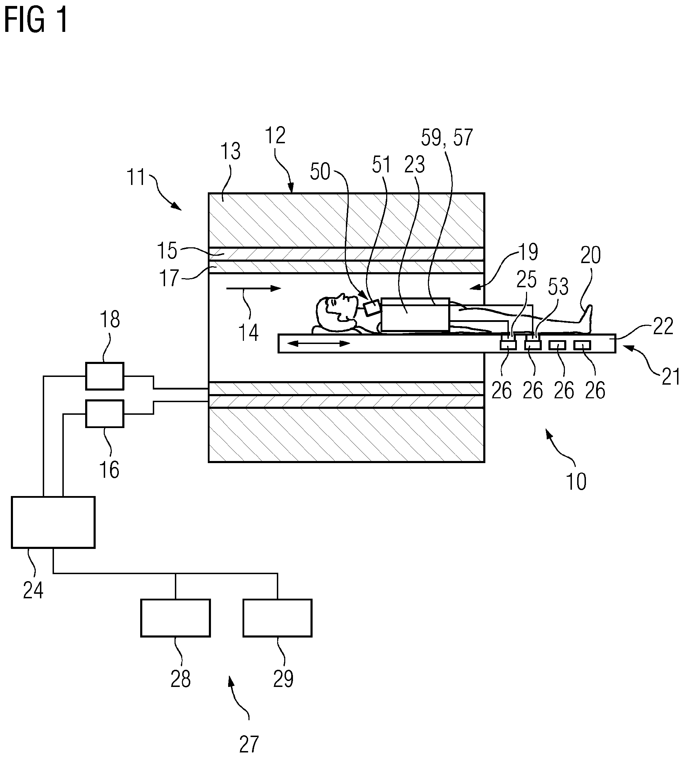

In Fig. 1 ist eine erfindungsgemäße Magnetresonanz-Elastographie-Vorrichtung 10 schematisch dargestellt. Die Magnetresonanz-Elastographie-Vorrichtung 10 umfasst eine Magnetresonanz-Einheit 11 und eine Elastographie-Einheit 50.

Die Magnetresonanz-Einheit 11 umfasst eine von einer Magneteinheit gebildeten Scannereinheit 12, die einen supraleitenden Grundmagneten 13 zu einem Erzeugen eines starken und insbesondere konstanten Grundmagnetfelds 14 umfasst. Die Scannereinheit 12 weist weiterhin eine Gradientenspuleneinheit 15 zu einer Erzeugung von Magnetfeldgradienten auf, die für eine Ortskodierung während einer Bildgebung verwendet werden. Die Gradientenspuleneinheit wird mittels einer Gradientensteuereinheit 16 der Magnetresonanz-Einheit 11 gesteuert. Die Scannereinheit 12 umfasst weiterhin eine Hochfrequenzspuleneinheit 17 zu einer Anregung einer Polarisation, die sich in dem von dem Grundmagneten erzeugten Grundmagnetfeld einstellt. Die Hochfrequenzspuleneinheit 17 wird von einer Hochfrequenzspulensteuereinheit 18 der Magnetresonanz-Einheit 11 gesteuert und strahlt hochfrequente Magnetresonanzsequenzen in einen Untersuchungsraum, der im Wesentlichen von einem Patientenaufnahmebereich 19 der Magnetresonanz-Einheit 11 gebildet ist, der Magnetresonanz-Einheit 11 ein. Die Hochfrequenzspuleneinheit 17 ist dabei fest innerhalb der Scannereinheit 12 integriert.

Zudem weist die Magnetresonanz-Einheit 11 den Patientenaufnahmebereich 19 auf zu einer Aufnahme eines Patienten 20. Der Patientenaufnahmebereich 19 im vorliegenden Ausführungsbeispiel ist zylinderförmig ausgebildet und in einer Umfangsrichtung von der Scannereinheit 12 zylinderförmig umgeben. Grundsätzlich ist jedoch eine davon abweichende Ausbildung des Patientenaufnahmebereichs 19 jederzeit denkbar. Der Patient 20 kann mittels einer Patientenlagerungsvorrichtung 21 der Magnetresonanz-Einheit 11 in den Patientenaufnahmebereich 19 geschoben und/oder gefahren werden. Die Patientenlagerungsvorrichtung 21 weist hierzu einen innerhalb des Patientenaufnahmebereichs 19 bewegbar ausgestalteten Patiententisch 22 auf.

Die Magnetresonanz-Einheit 11 umfasst des Weiteren zumindest eine lokale Hochfrequenzspule 23, die zu einem Empfang von Magnetresonanzsignalen während einer Magnetresonanzuntersuchung ausgelegt ist. Die lokale Hochfrequenzspule 23 an dem zu untersuchenden Körperbereich des Patienten 20 für eine Magnetresonanzuntersuchung angelegt. Im vorliegenden Ausführungsbeispiel ist die lokale Hochfrequenzspule 23 von einer Körperspule gebildet. Grundsätzlich sind auch weitere Ausgestaltungen der lokalen Hochfrequenzspule 23 in einer alternativen Ausgestaltung der Hochfrequenzspulen 23 jederzeit denkbar, wie beispielsweise eine Kopfspule oder eine Nackenspule oder eine Kniespule usw.

Für eine Übertragung der empfangenen Magnetresonanzsignale von der lokalen Hochfrequenzspule 23 an die Magnetresonanz-Einheit 11, insbesondere an eine Steuerungseinheit 24 der Magnetresonanz-Einheit 11, weist die lokale Hochfrequenzspule 23 ein Steckerelement 25, insbesondere einen Spulenstecker, auf. Das Steckerelement 25, insbesondere der Spulenstecker, der lokalen Hochfrequenzspule 23 ist mit einem Spulensteckerelement 26 Magnetresonanz-Einheit 11 kompatibel ausgebildet. Das Spulensteckerelement 26 ist insbesondere zur Aufnahme von dem Steckerelement 25, insbesondere dem Spulenstecker, der lokalen Hochfrequenzspule 23 ausgelegt. Bevorzugt ist das Spulensteckerelement 26 an der Patientenlagerungsvorrichtung 21 angeordnet und über die Patientenlagerungsvorrichtung 21 mit der Steuerungseinheit 24 der Magnetresonanz-Einheit 11 verbunden. Bevorzugt umfasst die Patientenlagerungsvorrichtung 21 zwei oder mehr Spulensteckerelemente 26 zu einer Anbindung und/oder Kopplung von zwei oder mehr lokalen Hochfrequenzspulen gleichzeitig während einer MagnetresonanzUntersuchung. Von der Patientenlagerungsvorrichtung 21 werden die Magnetresonanzsignale mittels einer nicht näher dargestellten Datenübertragungseinheit der Magnetresonanz-Einheit 11 an die Steuerungseinheit 24 übertragen.

Zu einer Steuerung des Grundmagneten 13, der Gradientensteuereinheit 16 und zur Steuerung der Hochfrequenzspulensteuereinheit 18 weist die Magnetresonanz-Einheit 11 die Steuerungseinheit 24 auf. Die Steuerungseinheit 24 steuert zentral die Magnetresonanz-Einheit 11, wie beispielsweise das Durchführen einer vorbestimmten bildgebenden Gradientenechosequenz. Zudem umfasst die Steuerungseinheit 24 eine nicht näher dargestellte Auswerteeinheit zu einer Auswertung von medizinischen Bilddaten, die während der Magnetresonanzuntersuchung erfasst werden.

Des Weiteren umfasst die Magnetresonanz-Einheit 11 eine Benutzerschnittstelle 27, die mit der Steuerungseinheit 24 verbunden ist. Steuerinformationen wie beispielsweise Bildgebungsparameter, sowie rekonstruierte Magnetresonanzbilder können auf einer Anzeigeeinheit 28, beispielsweise auf zumindest einem Monitor, der Benutzerschnittstelle 27 für ein medizinisches Bedienpersonal angezeigt werden. Weiterhin weist die Benutzerschnittstelle 27 eine Eingabeeinheit 29 auf, mittels der Informationen und/oder Parameter während eines Messvorgangs von dem medizinischen Bedienpersonal eingegeben werden können.

Die dargestellte Magnetresonanz-Einheit 11 kann selbstverständlich weitere Komponenten umfassen, die Magnetresonanz-Einheiten 11 gewöhnlich aufweisen. Eine allgemeine Funktionsweise einer Magnetresonanz-Einheit 11 ist zudem dem Fachmann bekannt, so dass auf eine detaillierte Beschreibung der weiteren Komponenten verzichtet wird.

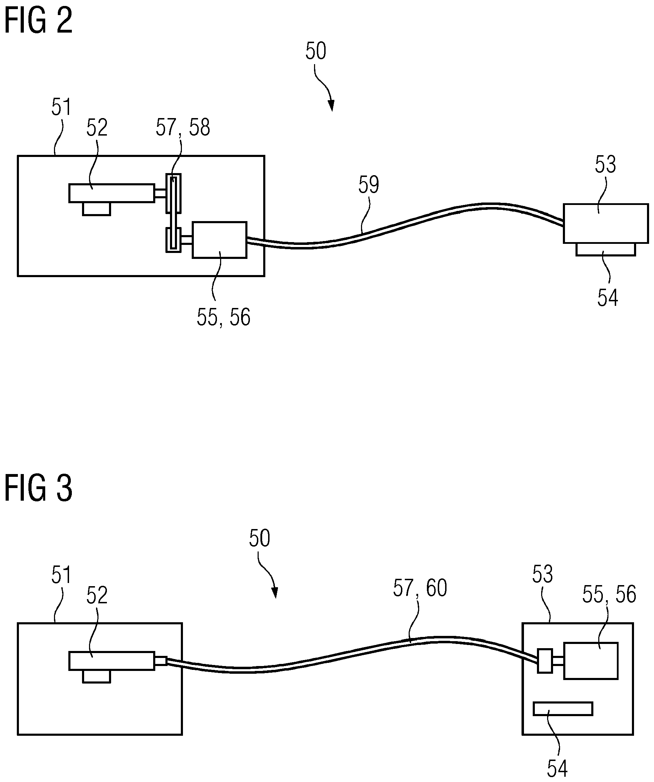

Die Elastographie-Einheit 50 der Magnetresonanz-Elastographie-Vorrichtung 10 umfasst einen Vibrationsapplikator 51, der eine Vibrationserzeugereinheit 52 aufweist (Fig. 2 und 3). Der Vibrationsapplikator 51 ist während einer Magnetresonanz-Elastographie-Untersuchung an einem Patienten 20 direkt am Patienten 20, insbesondere an dem zu untersuchenden Bereich des Patienten 20, angeordnet.

Des Weiteren weist die Elastographie-Einheit 50 eine Kopplungseinheit 53 zu einer Kopplung und/oder zu einer Verbindung der Elastographie-Einheit 50 mit der Magnetresonanz-Einheit 11 auf. Die Kopplungseinheit 53 weist ein Steckerelement 54 auf, das im vorliegenden Ausführungsbeispiel mit einem der Spulensteckerelemente der Magnetresonanz-Einheit 11, insbesondere der Patientenlagerungsvorrichtung 21 der Magnetresonanz-Einheit 11, kompatibel ausgebildet ist (Fig. 2 und 3). Eine Ansteuerung der Elastographie-Einheit 50 erfolgt somit mittels der Steuerungseinheit 24 der Magnetresonanz-Einheit 11, wobei die Steuersignale von der Magnetresonanz-Einheit 11, insbesondere von der Steuerungseinheit 24, über die Kopplungseinheit 53 an die Elastographie-Einheit 50 übertragen werden. Zudem erfolgt mittels der Kopplungseinheit 53, insbesondere des Steckerelements 54, der Elastographie-Einheit 50 eine Energieversorgung der Elastographie-Einheit 50 in einem gesteckten Zustand der Kopplungseinheit 53, insbesondere des Steckerelements 54.

Des Weiteren weist die Elastographie-Einheit 50 eine Antriebseinheit 55 auf. Die Antriebseinheit 55 ist dazu ausgelegt, ein Antriebsmoment für die Vibrationserzeugereinheit 52 während einer Elastographie-Untersuchung zu erzeugen und/oder zu generieren. Die Antriebseinheit 55 ist dabei magnetresonanzkompatibel ausgebildet. Dabei ist die Ausbildung der Antriebseinheit 55 derart, dass weder die Antriebseinheit 55 eine Magnetresonanzmessung beeinflusst noch mit dem Magnetfeld und/oder einem Gradientenfeld und/oder einem Hochfrequenzfeld der Magnetresonanz-Einheit 11 interagiert. Die magnetresonanzkompatible Antriebseinheit 55 umfasst bevorzugt eine Piezo-Antriebseinheit 56, bei der der piezoelektrischen Effekt zur Erzeugung und/oder Generierung eines Antriebsmoments nutzt wird (Fig. 2 und 3).

In Fig. 2 ist ein erstes Ausführungsbeispiel der Elastographie-Einheit 50 näher dargestellt. In diesem Ausführungsbeispiel ist die zu Fig. 1 beschriebene Antriebseinheit 55, insbesondere die Piezo-Antriebseinheit 56, innerhalb des Vibrationsapplikators 51 angeordnet. Aufgrund der magnetresonanzkompatiblen Ausbildung der Piezo-Antriebseinheit 56 ist somit eine Beeinflussung und/oder eine Interaktion mit dem Magnetfeld und/oder dem Gradientenfeld und/oder dem Hochfrequenzfeld der Magnetresonanz-Einheit 11 ausgeschlossen.

Die Elastographie-Einheit 50 in Fig. 2 weist zudem ein Kraftübertragungselement 57 auf, das eine Antriebskraft und/oder ein Antriebsmoment von der Antriebseinheit 55, insbesondere der Piezo-Antriebseinheit 56, auf die Vibrationserzeugereinheit 52 überträgt. Aufgrund der Anordnung der Antriebseinheit 55, insbesondere der Piezo-Antriebseinheit 56, innerhalb des Vibrationsapplikators 51 ist auch das Kraftübertragungselement 57 innerhalb des Vibrationsapplikators 51 angeordnet. Das Kraftübertragungselement 57 umfasst dabei eine Übertragungswelle 58. Durch die Anordnung der Antriebseinheit 55, insbesondere der Piezo-Antriebseinheit 56, innerhalb des Vibrationsapplikators 51 kann ein besonders kompaktes Kraftübertragungselement 57, insbesondere eine besonders kompakte Übertragungswelle 58, verwendet werden. Zudem kann das Kraftübertragungselement 57 in einer weiteren Ausgestaltung der Erfindung auch eine zu einer Übertragungswelle 58 alternative Ausbildung aufweisen, wie beispielsweise ein Zahnrad und/oder ein Getriebeelement usw.

Die Elastographie-Einheit 50 weist zudem eine elektrische Verbindung 59 auf, die zwischen der Kopplungseinheit 53 und dem Vibrationsapplikator 51 angeordnet ist, um Steuersignale von der Steuerungseinheit 24 der Magnetresonanz-Einheit 11 an den Vibrationsapplikator 51 zu übertragen und/oder eine Energieversorgung des Vibrationsapplikators 51 über die Magnetresonanz-Einheit 11 zu ermöglichen.

Die Kopplungseinheit 53 der in Fig. 2 dargestellten Elastographie-Einheit 50 ist entsprechend den Ausführungen zu der Kopplungseinheit 53 in Fig. 1 ausgebildet. Zudem ist auch eine Ausbildung der Vibrationserzeugereinheit 52 entsprechend den Ausführungen zu Fig. 1 ausgebildet.

In Fig. 3 ist ein alternatives Ausführungsbeispiel der Elastographie-Einheit 50 dargestellt. Im Wesentlichen gleich bleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 2, wobei bezüglich gleich bleibender Bauteile, Merkmale und Funktionen auf die Beschreibung des Ausführungsbeispiels in Fig. 2 verwiesen wird.

Die in Fig. 3 dargestellte Elastographie-Einheit 50 weist eine Antriebseinheit 55, insbesondere eine Piezo-Antriebseinheit 56 auf, die innerhalb der Kopplungseinheit 53 angeordnet ist. Hierdurch ist insbesondere der Vibrationsapplikator 51 besonders kompakt ausgeführt und ermöglicht somit einen hohen Patientenkomfort während einer Magnetresonanz-Elastographie-Untersuchung.

Zudem weist die Elastographie-Einheit 50 in Fig. 3 ein Kraftübertragungselement 57 auf. Im vorliegenden Ausführungsbeispiel überträgt das Kraftübertragungselement 57 eine Antriebskraft und/oder ein Antriebsmoment von der Antriebseinheit 55, insbesondere der Piezo-Antriebseinheit 56, innerhalb der Kopplungseinheit 53 an die Vibrationserzeugereinheit 52 innerhalb des Vibrationsapplikators 51. Das Kraftübertragungselement 57 ist somit zwischen der Kopplungseinheit 53 und dem Vibrationsapplikator 51 angeordnet. Das Kraftübertragungselement 57 weist eine Übertragungswelle 60 auf, wobei im vorliegenden Ausführungsbeispiel das Kraftübertragungselement 57, insbesondere die Übertragungswelle 60, eine flexible Übertragungswelle 60, wie beispielsweise eine Biegewelle, aufweist. Die flexible Übertragungswelle 60 ist insbesondere biegbar und/oder biegsam ausgebildet, so dass der Vibrationsapplikator 51 bezüglich der Kopplungseinheit 53 flexibel am Patienten 20 positioniert werden kann.

Das Steckerelement 54 der Kopplungseinheit 53 der in Fig. 2 dargestellten Elastographie-Einheit 50 ist entsprechend den Ausführungen zu der Kopplungseinheit 53 in Fig. 1 ausgebildet. Zudem ist auch eine Ausbildung der Vibrationserzeugereinheit 52 entsprechend den Ausführungen zu Fig. 1 ausgebildet.

Die in den Fig. 1 bis 3 dargestellten Elastographie-Einheiten 50 können selbstverständlich weitere Komponenten umfassen, die Elastographie-Einheiten 50 gewöhnlich aufweisen. Eine allgemeine Funktionsweise einer Elastographie-Einheit 50 ist zudem dem Fachmann bekannt, so dass auf eine detaillierte Beschreibung der weiteren Komponenten verzichtet wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanz-Elastographie-Vorrichtung umfassend:
- eine Magnetresonanz-Einheit, die einen Grundmagneten, eine Gradientenspuleneinheit und eine Hochfrequenzspuleneinheit aufweist, und
- eine Elastographie-Einheit mit einem Vibrationsapplikator, der eine Vibrationserzeugereinheit aufweist, und einer Kopplungseinheit zum Koppeln der Elastographie-Einheit mit der Magnetresonanz-Einheit,
**dadurch gekennzeichnet, dass** die Elastographie-Einheit eine Antriebseinheit zum Erzeugen eines Antriebmoments für die Vibrationserzeugereinheit aufweist, wobei die Antriebseinheit magnetresonanzkompatibel ausgebildet ist.

2. Magnetresonanz-Elastographie-Vorrichtung nach Anspruch 1,
**dadurch gekennzeichnet, dass** die Antriebseinheit eine Piezo-Antriebseinheit umfasst.

3. Magnetresonanz-Elastographie-Vorrichtung nach einem der vorhergehenden Ansprüche,
**dadurch gekennzeichnet, dass** die Antriebseinheit innerhalb des Vibrationsapplikators angeordnet ist.

4. Magnetresonanz-Elastographie-Vorrichtung nach einem der vorhergehenden Ansprüche,
**dadurch gekennzeichnet, dass** die Antriebseinheit innerhalb der Kopplungseinheit angeordnet ist.

5. Magnetresonanz-Elastographie-Vorrichtung nach einem der vorhergehenden Ansprüche,
**dadurch gekennzeichnet, dass** die Elastographie-Einheit ein Kraftübertragungselement aufweist, das ein von der Antriebseinheit erzeugtes Antriebsmoment auf die Vibrationserzeugereinheit überträgt.

6. Magnetresonanz-Elastographie-Vorrichtung nach Anspruch 5,
**dadurch gekennzeichnet, dass** das Kraftübertragungselement eine Übertragungswelle umfasst.

7. Magnetresonanz-Elastographie-Vorrichtung nach einem der Ansprüche 5 bis 6,
**dadurch gekennzeichnet, dass** das Kraftübertragungselement eine flexible Übertragungswelle aufweist.

8. Magnetresonanz-Elastographie-Vorrichtung nach einem der vorhergehenden Ansprüche,
**dadurch gekennzeichnet, dass** die Kopplungseinheit ein Steckerelement aufweist, das mit einem Spulensteckerelement der Magnetresonanz-Einheit zur Aufnahme eines Spulensteckers einer lokalen Hochfrequenzspule kompatibel ausgebildet ist.

9. Magnetresonanz-Elastographie-Vorrichtung nach einem der vorhergehenden Ansprüche,
**dadurch gekennzeichnet, dass** die Elastographie-Einheit eine elektrische Verbindung zwischen der Kopplungseinheit und dem Vibrationsapplikator aufweist.
